(19) Europäisches Patentamt
European Patent Office
Office européen des brevets

(11) Veröffentlichungsnummer: **0 001 402**
**A1**

(12) **EUROPÄISCHE PATENTANMELDUNG**

(21) Anmeldenummer: **78100868.5**

(22) Anmeldetag: **12.09.78**

(51) Int. Cl.²: **C 07 D 211/66**, C 07 D 221/20, C 07 D 471/10, C 07 D 211/94, C 08 K 5/34 // (C07D471/10, 221/00, 221/00)

(30) Priorität: **22.09.77 DE 2742582**

(43) Veröffentlichungstag der Anmeldung: **18.04.79 Patentblatt 79/8**

(84) Benannte Vertragsstaaten: **BE CH DE FR GB NL**

(71) Anmelder: **HOECHST AKTIENGESELLSCHAFT, Postfach 80 03 20, D-6230 Frankfurt/Main 80 (DE)**

(72) Erfinder: **Wiezer, Hartmut, Dr., Hans-Fischer-Strasse 6, D-8906 Gersthofen (DE)**
Erfinder: **Pfahler, Gerhard, Dr., Karlsbader Strasse 27, D-8900 Augsburg (DE)**
Erfinder: **Mayer, Norbert, Dr., Ziegelgrundweg 17, D-8901 Gablingen (DE)**

(54) **Substituierte Piperidinhydroxyamide, ihre Herstellung und Verwendung als Lichtschutzmittel.**

(57) Substituierte Piperidinhydroxyamide, ihre Herstellung und Verwendung als Lichtschutzmittel. Piperidinhydroxyamide der Formel

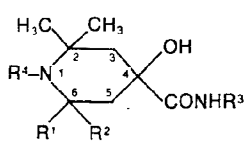

in der

$R^1$ und $R^2$ gleiche oder verschiedene geradkettige oder verzweigte Alkylreste mit 1 bis 12 C-Atomen sind, oder $R^1$ oder $R^2$ zusammen mit dem Kohlenstoffatom, an das sie gebunden sind, einen gegebenenfalls methylsubstituierten Cyclopentan- oder Cyclohexanring oder einen 2,2,6,6-Tetramethylpiperidinring, dessen Kohlenstoffatom 4 mit dem Kohlenstoffatom 6 des Piperidinhydroxyamidringes identisch ist, bilden,

$R^3$ für einen geradkettigen oder verzweigten Alkylrest mit 1 bis 18 C-Atomen, eine Hydroxymethylengruppe, oder einen gegebenenfalls durch einen $C_1$- bis $C_4$-Alkylrest substituierten Benzylrest steht, und

$R^4$ Wasserstoff, Sauerstoff, Hydroxyl, Hydroxymethylen oder eine $C_1$- bis $C_4$-Alkylgruppe ist,

sowie ein Verfahren zur Herstellung dieser Verbindungen.

Die Produkte werden – gegebenenfalls in Form von Salzen – zur Stabilisierung von Thermoplasten gegen den schädigenden Einfluß von Licht verwendet.

EP 0 001 402 A1

HOECHST AKTIENGESELLSCHAFT HOE 77/F 815 Dr.Mb/hka

Substituierte Piperidinhydroxyamide, ihre Herstellung und Verwendung als Lichtschutzmittel

Die Erfindung betrifft neue Derivate von Piperidinhydroxyamiden, deren Herstellung und ihre Verwendung als Stabilisatoren für synthetische Polymere.

Die neuen Verbindungen entsprechen der allgemeinen Formel (I)

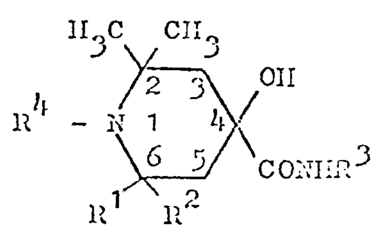

(I)

in der

$R^1$ und $R^2$ gleiche oder verschiedene geradkettige oder verzweigte Alkylreste mit 1 bis 12, vorzugsweise 1 bis 6, C-Atomen und insbesondere jedoch Methylgruppen sind, oder $R^1$ und $R^2$ zusammen mit dem Kohlenstoffatom, an das sie gebunden sind, einen gegebenenfalls methylsubstituierten Cyclopentan- oder Cyclohexanring oder einen 2.2.6.6-Tetramethylpiperidinring, dessen Kohlenstoffatom 4 mit dem

Kohlenstoffatom 6 des Piperidinhydroxyamidringes identisch ist, vorzugsweise letztgenannten, bilden,

$R^3$ für einen geradkettigen oder verzweigten Alkylrest mit 1 bis 18 C-Atomen, vorzugsweise einen Isoalkylrest mit 1 bis 12 C-Atomen und insbesondere einen tert.-Alkylrest mit 1 bis 8 C-Atomen, dessen tertiäres Kohlenstoffatom an den Amidstickstoff gebunden ist, eine Hydroxymethylengruppe, oder für einen gegebenenfalls durch einen $C_1$- bis $C_4$-Alkylrest substituierten Benzylrest steht, und

$R^4$ Wasserstoff, Sauerstoff, Hydroxyl, Hydroxymethylen oder eine $C_1$- bis $C_4$-Alkylgruppe ist.

Wenn das Stickstoffatom 6 mit H oder Alkyl substituiert ist, weist es basische Eigenschaften auf; die Verbindungen können in diesen Fällen auch in Form von Salzen mit anorganischen und organischen Säuren vorliegen.

Beispiele für die Reste $R^1$ und $R^2$ sind z.B. Methyl, Äthyl, i-Butyl, für Reste, in denen $R^1$ und $R^2$ zusammen mit dem Kohlenstoffatom, an das sie gebunden sind, einen Ring bilden, z.B. Cyclopentyl, Cyclohexyl oder 2.2.6.6-Tetramethylpiperidyl.

Beispiele für $R^3$ sind tert.-Butyl, 1.1-Dimethylpropyl, 1-Methyl-1-äthylpropyl, 1.1-Diäthylpropyl, 1.1.3.3-Tetramethylbutyl, Hydroxymethylen, Benzyl.

Beispiele für $R^4$ sind Wasserstoff, Hydroxymethylen, Methyl, Sauerstoff.

Beispiele für Salze der Verbindungen der Formel (I) sind Salze mit anorganischen Säuren wie Phosphate, Phosphite, Chloride, Sulfate und Salze mit organischen Mono- und Polycarbonsäuren wie Acetate, Laurate, Stearate, Succinate, Sebacate, Maleate, Citrate, Tartrate, Oxalate, Benzoate, Sulfonate, Phosphonate etc.

Von unter die allgemeine Formel (I) fallenden Verbindungen seien folgende beispielhaft aufgeführt:

2.2.6.6-Tetramethyl-4-hydroxy-4-tert.-butylcarbamoyl-piperidin

2.2.6.6-Tetramethyl-4-hydroxy-4-(1.1-dimethylpropyl)-carbamoylpiperidin

2.2.6.6-Tetramethyl-4-hydroxy-4-(1-methyl-1-äthyl-propyl)-carbamoylpiperidin

2.2.6.6-Tetramethyl-4-hydroxy-4-hydroxymethylencarbamoyl-piperidin

1-Hydroxymethylen-2.2.6.6-tetramethyl-4-hydroxy-4-hydroxy-methylencarbamoylpiperidin

D i e neuen Verbindungen der Formel (I), in denen beide Reste $R^3$ und $R^4$ keine Hydroxymethylengruppen sind, werden durch Umsetzen von 2.2-Dimethyl-6.6-dialkyl-4-hydroxy-4-cyanopiperidin mit einem Alkohol, gegebenenfalls aber auch einem Olefin erhalten. Den Reaktionsablauf gibt die nachstehende Gleichung (a) wieder, wobei die Reste $R^1$, $R^2$ und $R^3$ die früher angegebene Bedeutung haben.

(a)

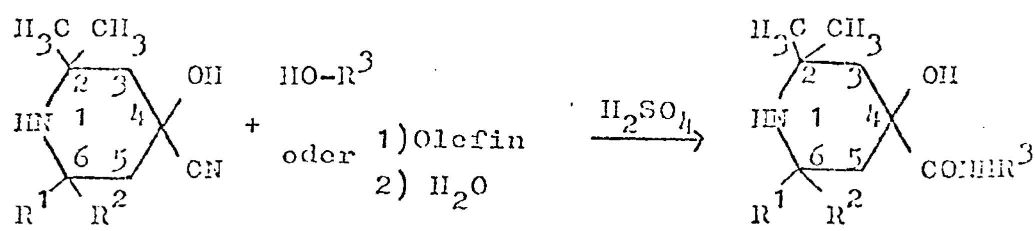


Die Umsetzung wird in diesem Falle in der Weise durchgeführt, dass man das Piperidincyanhydrin entweder in einem Überschuss des Alkohols oder zusammen mit der äquimolaren Menge des Alkohols oder Olefins in einem geeigneten organischen Lösungsmittel wie z.B. Eisessig vorlegt, die doppelt molare Menge Schwefelsäure zutropft und bei einer Temperatur von 30° bis 120° C, vorzugsweise von 50° bis 100° C und insbesondere von 60° bis 80° C reagieren lässt. Die Reaktionszeit beträgt 1 bis 20, vorzugsweise 2 bis 10 Stunden. Wird ein Olefin eingesetzt, so muss nach Beendigung der Umsetzung eine mindestens äquimolare Menge Wasser zugesetzt werden. Das im Verlaufe der Umsetzung ausgefallene Hydrosulfat - manchmal muss dasselbe durch Zusetzen eines Lösungsmittels wie z.B. Aceton ausgefällt werden - wird in wässriger Lösung durch Behandeln mit einer Base wie z.B. Ammoniak oder NaOH in die freie Base übergeführt und kann in dieser Form als Feststoff von der wässrigen Phase abfiltriert werden.

Verbindungen, in denen $R^3$ für eine Hydroxymethylengruppe steht, werden entsprechend Gleichung (b) erhalten,

(b) 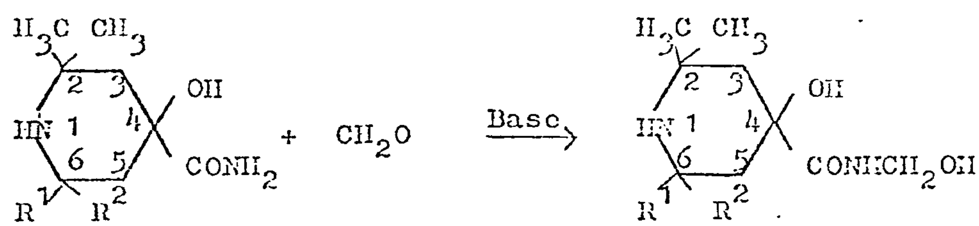


indem man ein an der Amidgruppe unsubstituiertes α-Hydroxyamid, das z.B. nach DE-OS 2,602,673 leicht zugänglich ist, in wässriger Lösung unter Zusatz einer starken Base wie z.B. $K_2CO_3$ als Katalysator bei Temperaturen von 20° bis 100° C, vorzugsweise 40° bis 80° C, mit Formaldehyd umsetzt. Bei Verwendung der doppelten Menge Formaldehyd wird auch die Aminogruppe des Piperidinringes durch Hydroxymethylen substituiert.

Die Verbindungen mit $R^4$ = Alkyl können hergestellt werden,

indem man die Verbindung mit $R^4$ = Wasserstoff in Gegenwart einer Base mit einem Alkylhalogenid umsetzt.

Die neuen Piperidinhydroxyamide eignen sich hervorragend zum Stabilisieren von synthetischen Polymeren gegen den Abbau durch Licht und Wärme, wobei unter synthetischen Polymeren in diesem Zusammenhang zu verstehen sind:

Halogenfreie und halogenhaltige Homo- und Copolymere, im einzelnen Homopolymerisate von Olefinen, Dienen und Styrol, wie z.B. Polyäthylen niedriger und hoher Dichte, Polypropylen, Polystyrol, Polybutadien und Polyisopren, Copolymere von Olefinen, Dienen und Styrol miteinander oder mit anderen olefinisch ungesättigten Monomeren, wie Äthylen-Propylen-Copolymere, Äthylen-Buten-Copolymere, Styrol-Butadien-Copolymere, Äthylen-Vinylacetat-Copolymere und Acrylnitril-Butadien-Copolymere, Homopolymere von Vinylchlorid und Vinylidenchlorid und Copolymere dieser Monomeren untereinander und mit anderen olefinisch ungesättigten Monomeren. Des weiteren sollen auch Polyurethane, Polyacetale, Polyester, Polyamide, Polyacrylate und Epoxyharze eingeschlossen sein. Bevorzugt sind Poly-α-Olefine wie Polyäthylene und insbesondere Polypropylene sowie die Polymeren des Vinylchlorids.

Überraschenderweise sind die erfindungsgemässen Verbindungen den strukturell ähnlichen Stabilisatoren nach den US-PSS 3,334,103 und 3,534,048, den FR-PSS 1,360,030 und 2,204,630, der JA-PS 71 31 733 und DE-OS 1,695-738 in ihrer Lichtschutzwirkung merklich überlegen, obwohl man eigentlich hätte annehmen müssen, dass auf Grund der an sich geringfügigen Veränderungen bezüglich der Struktur die Wirksamkeit in derselben Grössenordnung liegen sollte.

Die neuen stabilisierenden Verbindungen werden nach allgemein üblichen Methoden in die Polymermassen eingearbeitet. Alternativ kann man auch eine Lösung, Suspension oder Emulsion des Stabilisators mit dem Polymeren direkt, oder mit einer Lösung, Suspension oder Emulsion desselben vermischen und das Lösungsmittel anschliessend entfernen.

Die erfindungsgemässen Stabilisatoren sind für sich allein oder im Gemisch mit einem oder mehreren der bei der Kunststoffverarbeitung üblichen Stabilisatoren, wie z.B. Antioxidantien auf Phenol- und Sulfidbasis, UV-Absorbern und Lichtschutzmitteln, Phosphitstabilisatoren, Metallverbindungen, Epoxystabilisatoren und mehrwertigen Alkoholen einsetzbar. In den zu stabilisierenden Kunststoffmassen können ferner Flammschutzmittel und Pigmente, Farbstoffe, Antistatika und Füllstoffe wie z.B. Glasfasern anwesend sein.

Beispiele für geeignete Antioxidantien sind solche vom Typ der sterisch gehinderten Phenole wie 2,6-Di-t.-Butyl-p-kresol, 2,6-Di-octadecyl-p-kresol, 4,4'-Butyliden-bis-(2,6-di-t.-butyl-phenyl), 4,4'-Thio-bis-(2-t.-butyl-5-methylphenol), phenolische Triazinverbindungen, ferner Thiodipropionsäureester von Fettalkoholen, Dioctadecylsulfid und -disulfid.

Zu den UV-Absorbern und Lichtschutzmitteln gehören z.B. 2-(2'-Hydroxyphenyl)-benztriazole wie 2-(2'-Hydroxy-5'-methyl-phenyl)-bentriazol, 2-Hydroxybenzophenone wie 2-Hydroxy-4-octoxy-benzophenon, Stabilisatoren aus der Gruppe der Salizylate wie Octylphenylsalizylat, Nickelchelate, Oxalsäurediamide und sterisch gehinderte Piperidinverbindungen.

Als Phosphite sind Trisnonylphenylphosphit, Trislaurylphosphit oder auch Ester des Pentaerythritphosphits zu nennen.

Unter als Stabilisatoren bekannten Metallverbindungen werden in diesem Zusammenhang verstanden: Calcium-, Barium-,

Strontium-, Zink-, Cadmium-, Magnesium-, Aluminium- und Bleiseifen aliphatischer Carbonsäuren oder Oxycarbonsäuren mit etwa 12 bis 32 C-Atomen, Salze der genannten Metalle mit aromatischen Carbonsäuren wie Benzoate oder Salizylate sowie (Alkyl-)Phenolate dieser Metalle, ferner Organozinnverbindungen wie z.B. Dialkylzinnthioglykolate und Carboxylate.

Bekannte Epoxystabilisatoren sind z.B. epoxidierte höhere Fettsäuren wie epoxidiertes Sojabohnenöl, Tallöl, Leinöl oder epoxidiertes Butyloleat sowie Epoxide langkettiger Olefine.

Mehrwertige Alkohole können beispielsweise Pentaerythrit, Trimethylolpropan, Sorbit oder Mannit sein, d.h. bevorzugt Alkohole mit 5 oder 6 C-Atomen und 3 bis 6 OH-Gruppen.

Eine wirksame Stabilisatorkombination für Poly-α-Olefine, wie z.B. Hoch-, Mittel- und Niederdruckpolymerisate von $C_2$- bis $C_4$-α-Olefinen, insbesondere Polyäthylen und Polypropylen oder von Copolymerisaten derartiger α-Olefine besteht, bezogen auf 100 Gewichtsteile Polymer, beispielsweise aus 0,01 bis 5 Gewichtsteilen einer der erfindungsgemäss zu verwendenden Verbindungen, 0,05 bis 5 Gewichtsteilen eines phenolischen Stabilisators, gegebenenfalls 0,01 bis 5 Gewichtsteilen eines schwefelhaltigen Costabilisators sowie gegebenenfalls 0,01 bis 3 Gewichtsteilen einer basischen oder neutralen Metallseife, wie z.B. Calciumstearat oder Zinkstearat, sowie gegebenenfalls 0,1 bis 5 Gewichtsteilen eines Phosphits und gegebenenfalls 0,01 bis 5 Gewichtsteilen eines bekannten UV-Stabiliators aus der Gruppe der Alkoxyhydroxybenzophenone, Hydroxyphenylbenzotriazole, Benzylidenmalonsäuremononitrilester oder der sog. Quencher wie z.B. Nickelchelate.

Die folgenden Beispiele dienen der näheren Erläuterung der Erfindung. Die Strukturen der Verbindungen wurden kernresonanzspektroskopisch ermittelt.

Beispiel 1

2.2.6.6-Tetramethyl-4-hydroxy-4-hydroxymethylencarbamoyl-piperidin

116 g 2.2.6.6-Tetramethyl-4-hydroxy-4-carbamoylpiperidin werden in 70 ml Wasser vorgelegt, worauf man 45 g 40 %ige Formaldehydlösung und 2 g $K_2CO_3$ zugibt, das Gemisch innerhalb von 2 Stunden auf 80° C aufheizt und 1 Stunde bei 80° C weiterrührt. Der Reaktionsansatz wird sodann auf 1/4 seines Volumens eingeengt, dann nutscht man den Feststoff ab und trocknet. Ausbeute 66 g, Fp. 92° C

Beispiel 2

1-Hydroxymethylen-2.2.6.6-tetramethyl-4-hydroxy-4-hydroxy-methylencarbamoylpiperidin

20 g 2.2.6.6-Tetramethyl-4-hydroxy-4-carbamoylpiperidin werden in 20 ml Wasser vorgelegt. 30 g 40 %ige Formaldehydlösung und 0,5 g $K_2CO_3$ werden zugegeben, worauf man das Gemisch 4 Stunden unter Rühren auf 100° C erhitzt. Nach dem Abdestillieren des Wassers wird der Rückstand mit Aceton verrührt, wobei sich das gewünschte Produkt abscheidet. Ausbeute 16 g, Fp. 164° C

Beispiel 3

2.2.6.6-Tetramethyl-4-hydroxy-4-tert.-butylcarbamoyl-piperidin

54,6 g 2.2.6.6-Tetramethyl-4-hydroxy-4-cyanopiperidin werden zusammen mit 45 g tert.-Butanol in 200 ml Eisessig vorgelegt. Dann werden unter Rühren 60 g konz. Schwefelsäure zugetropft, worauf man 2 Stunden bei 70° C weiterrührt, wobei sich ein Niederschlag bildet, der abfiltriert und in Wasser gelöst wird. Beim Alkalisieren mit NaOH fällt das Verfahrensprodukt aus, welches abgenutscht und aus Essigester umkristallisiert wird. Ausbeute 44 g, Fp. 134° bis 136° C

Beispiel 4

2.2.6.6-Tetramethyl-4-hydroxy-4-(1.1-dimethylpropylcarbamoyl)-piperidin

wird analog Beispiel 3, jedoch unter Verwendung von 2-Methyl-butanol-2 anstelle von tert.-Butanol hergestellt und aus Heptan umkristallisiert. Ausbeute 55 %, Fp. 115° bis 117° C

Beispiel 5

2.2.6.6-Tetramethyl-4-hydroxy-4-(1-methyl-1-äthylpropylcarbamoyl)-piperidin

36,4 g 2.2.6.6-Tetramethyl-4-hydroxy-cyanopiperidin werden in 100 ml 3-Methylpentanol-3 vorgelegt. Unter Rühren werden 40 g konz. Schwefelsäure zugetropft, worauf man 1 Stunde auf 70° C erwärmt. Der ausgefallene Niederschlag wird sodann abgenutscht und in Wasser mit NaOH behandelt. Man gewinnt die freie Base durch Filtration und kristallisiert aus Essigester um. Ausbeute 38 g, Fp. 127° bis 129° C

Beispiel 6

Dieses Beispiel zeigt die lichtstabilisierende Wirkung der erfindungsgemässen Verbindungen beim Einsatz in einem Poly-α-Olefin.

100 Gewichtsteile Polypropylen mit einem Schmelzindex $i_5$ von ca. 6 g/10 min. (bestimmt nach ASTM D 1238-62 T) und einer

Dichte von 0,96 wurden mit

0,1 Gew.-Teilen Pentaerythrityl-tetrakis-[3-(3,5-di-tert.-butyl-4-hydroxyphenyl)-propionat],

0,2 Gew.-Teilen Calciumstearat und

0,1 Gew.-Teilen des zu prüfenden erfindungsgemässen Stabilisators

vermischt.

Um eine möglichst gleichmässige Verteilung auf dem Polymerkorn zu erreichen, wurden die Stabilisatoren in einem Lösungsmittel gelöst und die Lösung wurde unter Rühren in das Polypropylenpulver eingetropft, wobei durch gleichzeitige Bestrahlung mit einer IR-Lampe der grösste Teil des Lösemittels wieder abdampfte. Nach ca. 20 Minuten wurde das Calciumstearat hinzugegeben und noch weitere 10 Minuten gemischt. Lösemittelreste wurden durch Trocknen bei 50° C/ 120 min. im Trockenschrank entfernt.

Die stabilisierte Kunststoffmischung wurde auf einer Windsor-Spritzgussmaschine der Type SP 50 bei 250° C zu 60 x 60 x 1 mm-Platten verspritzt. Aus diesen Platten wurden Prüfkörper nach DIN 53 455 Form 3, verkleinert im Maßstab 1 : 3, ausgestanzt. Die als Vergleichsmuster benötigten Prüfkörper wurden analog, jedoch unter Fortlassen des zu testenden Stabilisators (Versuch g) bzw. unter Verwendung bekannter Lichtstabilisatoren (Versuche c bis f), hergestellt.

Die Prüfung der Lichtbeständigkeit wurde in einer Xenotest-450-Apparatur der Firma Original Hanau Quarzlampen GmbH mit der Filterkombination 6 IR + 1 UV gemäss DIN 53 387 "Kurzprüfung der Wetterbeständigkeit" vorgenommen.

Während der Belichtungszeit betrug die Schwarztafeltemperatur 43° C ± 1° C, die relative Luftfeuchtigkeit im Probenraum 70 % ± 1 %. Der Probenraum wurde alle 2 Stunden 5 Minuten lang mit Frischluft gespült. Die Reissdehnung wurde auf einer Zugprüfmaschine der Firma Instron bei einer Abzugsgeschwindigkeit von 5 cm/min. nach einer definierten Belichtungszeit ermittelt. Die Ergebnisse sind in der nachstehenden Tabelle zusammengestellt.

Der Stabilitätsfaktor ergibt sich aus dem Verhältnis der Bestrahlungszeit des stabilisierten Probekörpers, wobei jeweils so lange bestrahlt wurde, bis die Reissdehnung auf die Hälfte des Ausgangswertes gefallen ist.

Die stabilisierende Wirkung der erfindungsgemässen Verbindungen ist besser als die eines Benzophenon- bzw. Benzotriazol-Stabilisators. Sie ist auch besser als die des 2.2.6.6-Tetramethyl-4-hydroxy-4-cyanopiperidins (US-PS 3,334,103, FR-PS 1,360,030 und JA-PS 71 31 733) und des 2.2.6.6-Tetramethyl-4-hydroxyiminopiperidins (JA-PS 71 31 733), von denen letzteres obendrein noch eine Trübung der Polymermassen verursacht.

| Versuchs-Nr. | Stabilisator nach Beispiel | Stabilitätsfaktor |
|---|---|---|
| a) | 2 | > 5 |
| b) | 3 | > 5 |
| c) | Benzophenon-Stabilisator 1) | < 2,5 |
| d) | Benzotriazol-Stabilisator 2) | < 2,5 |
| e) | 2.2.6.6-Tetramethyl-4-hydroxyiminopiperidin | 3,0 |
| f) | 2.2.6.6-Tetramethyl-4-hydroxy-4-cyano-piperidin | 2,8 |
| g) | Kontrolle (ohne Stabilisator) | 1 |

1) 2-Hydroxy-4-n-octyloxybenzophenon

2) 2-(2-Hydroxy-3'.5'-di-tert.-butylphenyl)-5-chlorbenzotriazol

Patentansprüche:

1. Piperidinhydroxyamide der allgemeinen Formel (I)

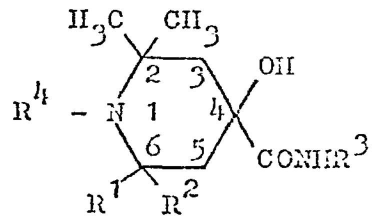

(I)

in der

$R^1$ und $R^2$ gleiche oder verschiedene geradkettige oder verzweigte Alkylreste mit 1 bis 12 C-Atomen sind, oder $R^1$ und $R^2$ zusammen mit dem Kohlenstoffatom, an das sie gebunden sind, einen gegebenenfalls methylsubstituierten Cyclopentan- oder Cyclohexanring oder einen 2.2.6.6-Tetramethylpiperidinring, dessen Kohlenstoffatom 4 mit dem Kohlenstoffatom 6 des Piperidinhydroxyamidringes identisch ist, bilden,

$R^3$ für einen geradkettigen oder verzweigten Alkylrest mit 1 bis 18 C-Atomen, eine Hydroxymethylengruppe, oder einen gegebenenfalls durch einen $C_1$- bis $C_4$-Alkylrest substituierten Benzylrest steht, und

$R^4$ Wasserstoff, Sauerstoff, Hydroxyl, Hydroxymethylen oder eine $C_1$- bis $C_4$-Alkylgruppe ist.

2. Verbindungen gemäss Anspruch 1, in denen $R^1$ und $R^2$ Methylgruppen sind und $R^4$ = H ist.

3. Verfahren zur Herstellung der Verbindungen nach den Ansprüchen 1 und 2, ausgenommen solchen, bei denen $R^3$ und/oder $R^4$ die Bedeutung von Hydroxymethylen hat, dadurch gekennzeichnet, dass man 2.2-Dimethyl-6.6-dialkyl-4-hydroxy-4-cyano-piperidin in einem organischen Lösungsmittel mit einem geradkettigen oder verzweigten Alkohol mit 1 bis 18 C-Atomen oder einem Olefin der gleichen

Kohlenstoffatomzahl unter Zusatz von Schwefelsäure als Katalysator umsetzt.

4. Verfahren zur Herstellung der Verbindungen nach den Ansprüchen 1 und 2 mit $R^3$ und/oder $R^4$ = Hydroxymethylen, dadurch gekennzeichnet, dass man ein 2.2-Dimethyl-6.6-dialkyl-4-hydroxy-carbamoylpiperidin unter Zusatz einer anorganischen Base als Katalysator in wässriger Lösung mit Formaldehyd umsetzt.

5. Verwendung der Verbindungen nach einem der Ansprüche 1 und 2, gegebenenfalls in Form des Salzes einer anorgani schen oder organischen Säure, zum Stabilisieren von syn thetischen Polymeren gegen den schädigenden Einfluss vo Licht.

6. Verwendung nach Anspruch 5, dadurch gekennzeichnet, das das Polymere ein Polyolefin ist.

7. Verwendung nach Anspruch 5, dadurch gekennzeichnet, das das Polymere ein halogenhaltiges Polymeres ist.

8. Verfahren zum Stabilisieren von synthetischen Polymeren gegen den schädigenden Einfluss von Licht, dadurch gekennzeichnet, dass man den Polymeren, gegebenenfalls neben bereits bekannten, stabilisierend wirkenden Stoffen, 0,01 bis 5 Gewichtsteile, bezogen auf Polymeres, einer Verbindung nach einem der Ansprüche 1 und 2 zusetzt wobei diese Verbindung auch in Form eines Salzes mit einer anorganischen oder organischen Säure vorliegen kann.

9. Gegen UV-Zersetzung stabilisierte, synthetische Polymere, in denen 0,01 bis 5 Gewichtsteile, bezogen auf das Polymere, einer Verbindung nach einem der Ansprüche 1 und 2, gegebenenfalls in Form des Salzes mit einer anorganischen oder organischen Säure, enthalten ist.

Europäisches Patentamt

# EUROPÄISCHER RECHERCHENBERICHT

0001402

Nummer der Anmeldung
EP 78 10 0868

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| X | FR - A - 2 351 102 (CIBA-GEIGY)<br>* Patentansprüche; Seite 9, Zeile 16 - Seite 10, Zeile 38; Seite 11, Zeile 36 - Seite 12 *<br>-- | 1-3,5, 6,8,9 | C 07 D 211/66<br>221/20<br>471/10<br>211/94<br>C 08 K 5/34//<br>(C 07 D 471/10<br>221/00<br>221/00) |
| | DE - A - 2 602 673 (HOECHST)<br>* Patentansprüche; Seite 5, Absatz 3; Seiten 7-8 *<br>-- | 1-3, 5-9 | RECHERCHIERTE SACHGEBIETE (Int. Cl.²) |
| | DE - A - 1 695 738 (SANKYO)<br>* Patentansprüche; Beispiel 8; Seiten 21-23; Beispiel 9, Seiten 23-24; Seiten 3-4 *<br>---- | 1-3,5, 6 | C 07 D 211/66<br>221/20<br>471/10<br>C 08 K 5/34 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 07-12-1978 | NUYTS |